(19) Europäisches Patentamt / European Patent Office / Office européen des brevets

(11) **EP 1 742 706 B1**

(12) **FASCICULE DE BREVET EUROPEEN**

(45) Date de publication et mention de la délivrance du brevet:
**21.08.2013 Bulletin 2013/34**

(21) Numéro de dépôt: **05737906.7**

(22) Date de dépôt: **28.04.2005**

(51) Int Cl.:
*A61N 7/02* (2006.01)    *A61N 1/40* (2006.01)

(86) Numéro de dépôt international:
**PCT/EP2005/051926**

(87) Numéro de publication internationale:
**WO 2005/107869 (17.11.2005 Gazette 2005/46)**

(54) **ENSEMBLE DE TRAITEMENT THERMIQUE DE TISSUS BIOLOGIQUES**

ANORDNUNG FÜR DIE WÄRMEBEHANDLUNG VON BIOLOGISCHEM GEWEBE

ASSEMBLY FOR HEAT TREATING BIOLOGICAL TISSUE

(84) Etats contractants désignés:
**AT BE BG CH CY CZ DE DK EE ES FI FR GB GR HU IE IS IT LI LT LU MC NL PL PT RO SE SI SK TR**

(30) Priorité: **29.04.2004 FR 0404562**

(43) Date de publication de la demande:
**17.01.2007 Bulletin 2007/03**

(73) Titulaire: **Koninklijke Philips Electronics N.V.
5656 AE Eindhoven (NL)**

(72) Inventeurs:
• **MOONEN, Chretien Theophilus Willibrordus
F-33170 GRADIGNAN (FR)**
• **SALOMIR, Rares
Chaponost 69630 (FR)**
• **MOUGENOT, Charles
F-33000 BORDEAUX (FR)**
• **PALUSSIERE, Jean
F-33000 BORDEAUX (FR)**

(74) Mandataire: **Damen, Daniel Martijn et al
Philips Intellectual
Property & Standards
High Tech Campus 44
5656 AE Eindhoven (NL)**

(56) Documents cités:
FR-A- 2 798 296        US-A1- 2003 019 681
US-A1- 2004 010 289    US-B1- 6 350 276
US-B1- 6 458 121

**Description**

L'invention concerne le domaine du traitement de tissus biologiques par hyperthermie.

Les thérapies par hyperthermie sont des techniques couramment utilisées pour traiter localement des tissus biologiques. Elles consistent à chauffer au moyen d'une source d'énergie (laser, micro-ondes, ondes radiofréquences, ultrasons) une zone cible du tissu biologique.

D'une manière générale, les thérapies par hyperthermie locale permettent des interventions médicales dont la nature invasive est réduite au minimum. Parmi les types d'énergie utilisés, les ultrasons focalisés (FUS) sont particulièrement intéressants puisqu'ils permettent de chauffer une zone cible, de manière non invasive et en profondeur dans les tissus.

Pendant le traitement, la température de la zone cible et de son environnement immédiat doit être contrôlée de manière précise et continue. Or l'élévation de température générée par les ultrasons dans les tissus est difficile à évaluer car cette élévation de température dépend de caractéristiques biologiques et physiologiques (absorption, diffusion de chaleur) du tissu dans la zone cible.

Le document FR 2 798 296 (publié le 16 mars 2001) décrit un ensemble pour le traitement thermique d'une zone cible d'un tissu biologique comprenant des moyens générateurs d'énergie pour fournir de l'énergie en un point focal fixe de la zone cible, des moyens pour mesurer la température dans la zone cible, et une unité de contrôle pour commander la quantité d'énergie fournie par les moyens générateurs.

Le document FR 2 823 678 (publié le 25 octobre 2002) décrit un ensemble pour le traitement thermique permettant de contrôler de manière automatique la température dans une région cible du tissu à traiter. L'ensemble comprend un générateur d'ultrasons, des moyens d'imagerie IRM pour mesurer et enregistrer la distribution spatiale de température dans la, région cible et une unité de contrôle comprenant des moyens de traitement numérique point par point de la distribution spatiale de température. L'unité de contrôle commande le déplacement dans l'espace du générateur d'ultrasons en fonction de la distribution de température mesurée par les moyens d'imagerie de manière à ce que la température dans la région cible suive un profil de température de consigne.

Le document «Local hyperthemia with MR-guided focused ultrasound : Spiral trajectory of the focal point optimized for temperature uniformity in the target region », Journal of magnetic résonance imaging 12 :571-583 (publié en 2000) décrit un procédé de traitement par ultrasons focalisés ayant pour but d'obtenir une élévation de température uniforme dans une région cible de volume important. Selon ce procédé, le point focal d'un générateur d'ultrasons est déplacé le long d'une première trajectoire en forme de spirale. La distribution spatiale de température dans la région cible est mesurée par IRM. En fonction de la distribution spatiale obtenue, une deuxième trajectoire en forme de spirale est déterminée, dans laquelle la vitesse de déplacement du point focal a été modifiée de manière à compenser des inhomogénéités de distribution de température persistantes après la première trajectoire. Le point focal du générateur d'ultrasons est déplacé le long de cette deuxième trajectoire.

Toutefois, un tel procédé est basé sur une modélisation linéaire du comportement des tissus. Or certains tissus peuvent présenter un comportement non-linéaire, en particulier en ce qui concerne leurs caractéristiques de conduction thermique. Il peut en résulter une instabilité de l'asservissement en température.

Un but de l'invention est de proposer un dispositif de traitement stable, présentant une bonne tolérance aux incertitudes d'estimation des paramètres physiologiques.

A cet effet, l'invention propose un ensemble de traitement thermique d'une région d'un tissu biologique comprenant :

- des moyens générateurs d'énergie pour fournir de l'énergie en un point focal dans la région,
- des moyens pour mesurer la distribution spatiale de température dans ladite région,
- une unité de contrôle apte à commander le déplacement du point focal le long d'une trajectoire prédéterminée en vue d'obtenir une distribution spatiale de température conforme à une distribution de consigne,

caractérisé en ce qu'au cours du déplacement du point focal, l'unité de contrôle est apte à commander la répartition d'énergie fournie par les moyens générateurs le long de la trajectoire, en fonction de la distribution de température mesurée et de la distribution de consigne, selon une loi de régulation comprenant un terme Proportionnel-Intégral-Dérivé.

La loi de régulation utilisée dans cet ensemble de traitement permet d'atteindre un profil de température de consigne prédéfini dans une région cible étendue, malgré d'éventuels effets physiologiques non linéaires lors du traitement.

Dans une mise en oeuvre de l'ensemble de traitement, l'unité de contrôle est apte à commander le déplacement du point focal le long d'une série de trajectoires successives et à commander pour chaque trajectoire une répartition d'énergie correspondante, en fonction d'une distribution de consigne associée à cette trajectoire et des distributions de température mesurées au cours des trajectoires précédentes.

Dans cette mise en oeuvre, l'unité de contrôle est apte à commander le déplacement du point focal selon une première trajectoire et à en déduire, en fonction de l'élévation de température mesurée, un coefficient de diffusion thermique D dans la région cible. L'unité de contrôle est apte à prendre en compte ce coefficient de diffusion thermique dans la loi de régulation.

Dans une mise en oeuvre de l'invention, pour commander une répartition d'énergie, l'unité de contrôle est apte à déterminer une fonction de répartition définissant la position du point focal le long de la trajectoire en fonction du temps.

Dans une mise en oeuvre de l'invention, l'unité de contrôle est apte à commander le déplacement du point focal en une pluralité de points de tir discrets répartis le long de la trajectoire. L' unité de contrôle est apte à commander les moyens générateurs d'énergie pour qu'ils déposent en chaque point de tir une quantité donnée d'énergie, égale d'un point de tir à l'autre. Dans cette mise en oeuvre, c'est la répartition des points de tir sur la trajectoire qui détermine la répartition de l'énergie déposée dans la région cible.

L'invention se rapporte également à un procédé de traitement thermique d'une région d'un tissu biologique dans lequel des moyens générateurs d'énergie fournissent de l'énergie en un point focal dans ladite région, des moyens de mesure mesurent la distribution spatiale de température dans ladite région et une unité de contrôle commande le déplacement du point focal le long d'une trajectoire prédéterminée en vue d'obtenir une distribution spatiale de température conforme à une distribution de consigne, caractérisé en ce qu'au cours du déplacement du point focal, l'unité de contrôle commande la répartition d'énergie fournie par les moyens générateurs le long de la trajectoire, en fonction de la distribution de température mesurée et de la distribution de consigne, selon une loi de régulation comprenant un terme Proportionnel-Intégral-Dérivé.

D'autres caractéristiques et avantages ressortiront encore de la description qui suit, laquelle est purement illustrative et non limitative et doit être lue en regard des figures annexées parmi lesquelles :

- la figure 1 représente de manière schématique un exemple d'ensemble de traitement thermique conforme à un mode de réalisation de l'invention,
- la figure 2 représente de manière schématique les étapes de traitement réalisées par l'ensemble de traitement thermique,
- la figure 3 représente des exemples a et b de profils spatiaux de température de consigne tels qu'ils peuvent être commandés dans le cas d'une trajectoire en forme de spirale,
- la figure 4 représente un exemple de trajectoire en forme de spirale elliptique parcourue par le point focal des moyens générateurs d'énergie,
- la figure 5 comprend deux diagrammes représentatifs respectivement de la distribution de température mesurée après que le point focal des moyens générateurs d'énergie ait parcouru une trajectoire en forme de spirale et de la distribution de température simulée pour un coefficient de diffusion thermique uniforme des tissus à traiter,
- la figure 6 représente une courbe de l'évolution de la température au centre de la région cible à traiter en fonction du temps au cours d'une série de dix trajectoires successives, la température de consigne étant de supérieure de 8°C par rapport à la température initiale, dans toute la région cible, lors d'une expérimentation ex vivo,
- la figure 7 représente l'évolution de paramètres au cours de chaque trajectoire de la série réalisée pour la figure 6, ces paramètres comprenant : une coordonnée du point focal suivant l'un des axes (Ox) du repère dans lequel est réalisée la trajectoire, la puissance ultrasonore déposée par les moyens générateurs d'énergie, le profil de température mesurée dans une coupe de la région cible et le profil de montée en température mesurée dans la coupe de la région cible,
- la figure 8 représente une courbe de l'évolution de la température au centre de la région cible à traiter en fonction du temps au cours d'une série de dix trajectoires successives, la température de consigne étant supérieure de 18°C par rapport à la température initiale, dans toute la région cible, lors d'une expérimentation ex vivo,
- la figure 9 représente l'évolution de paramètres au cours de chaque trajectoire de la série réalisée pour la figure 8, ces paramètres comprenant : une coordonnée du point focal suivant l'un des axes (Ox) du repère dans lequel est réalisée la trajectoire, la puissance ultrasonore déposée par les moyens générateurs d'énergie, le profil de répartition d'énergie dans une coupe de la région cible et le profil de montée en température mesurée dans la coupe de la région cible,
- la figure 10 est un exemple de carte de température tridimensionnelle obtenue par un appareil d'imagerie IRM,
- la figure 11 représente une courbe de l'évolution de la température au centre de la région cible à traiter en fonction du temps, lors d'une expérimentation in vivo,
- la figure 12 comprend trois diagrammes a, b et c représentant respectivement une trajectoire en spirale théorique comprend un tour supplémentaire s'étendant hors de la région cible à traiter, la projection des points de tir du tour supplémentaire sur le contour de la région cible, la trajectoire réelle comprenant les points de tir projeté du tour supplémentaire.

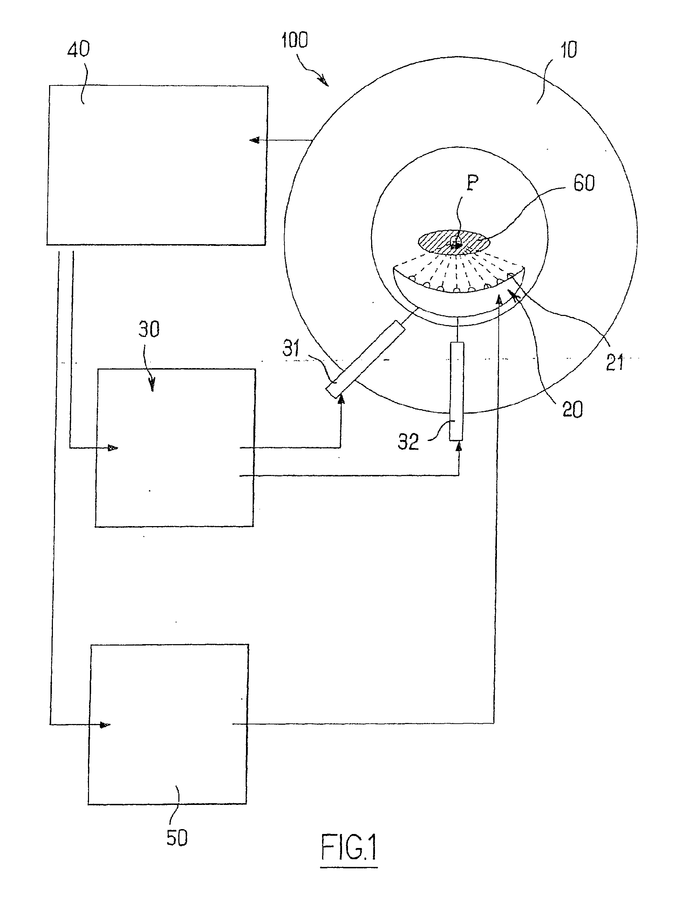

Sur la figure 1, l'ensemble de traitement 1 représenté comprend un appareil d'imagerie IRM comportant un aimant 10. L'ensemble 1 comprend des moyens générateurs d'énergie sous la forme d'une sonde annulaire 20 et d'un générateur multivoie 50 alimentant la sonde annulaire 20. La sonde 20 est intégrée dans le lit de l'aimant 10 et comporte des éléments générateurs 21 aptes à émettre des ultrasons en direction d'un point focal P lorsque la sonde est alimentée par le générateur 50.

La sonde annulaire 20 est par exemple une sonde fabriquée par la société Ultrasonic (Besançon, France) présentant un rayon de 80 mm, un diamètre d'ouverture de 96 mm et une variation de la distance focale comprise entre 60 et 110 mm suivant l'axe vertical. Les éléments générateurs d'ultrasons 21 sont aptes à émettre à une fréquence d'environ 1,5 MHz.

Les moyens générateurs d'énergie 20 peuvent être déplacés dans le plan horizontal au moyen d'un système de déplacement hydraulique 30 à pistons 31 et 32 fourni par la société LEP (Paris, France). Ce système de déplacement 30 dispose de moyens de guidage par laser et présente une vitesse maximale de déplacement de l'ordre de 3 mm par seconde, sa vitesse utile étant de 2 mm par seconde.

L'ensemble 1 comprend également une unité de contrôle 40 incluant une unité centrale qui est apte à recevoir en entrée des données en provenance de l'appareil d'imagerie IRM et est apte, en fonction de ces données, à commander le système de déplacement 30 pour modifier la position du point focal P de la sonde annulaire 20.

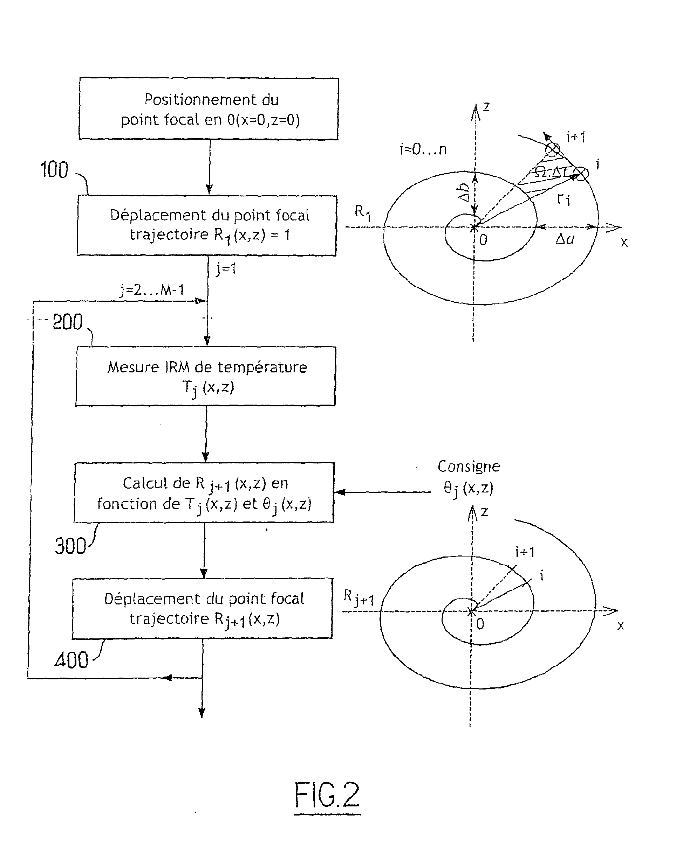

En fonctionnement, l'ensemble de traitement thermique 1 est utilisé pour traiter une région cible 60 de tissus d'un patient. L'unité de contrôle 40 commande le générateur multivoie 50 et le système de déplacement 30 pour réaliser les étapes représentées sur la figure 2.

Initialement, les moyens générateurs d'énergie 20 sont disposés par rapport au patient de sorte que le point focal P soit positionné en un point $O$ situé sensiblement au centre de la région cible 60 à traiter.

La figure 2 représente de manière schématique les étapes de traitement réalisées par l'ensemble de traitement thermique 1.

Selon une première étape 100, l'unité de contrôle 40 commande le système de déplacement 30 pour positionner le point focal des moyens générateurs d'énergie successivement en une pluralité de points de tir le long d'une première trajectoire prédéterminée (j=1). La trajectoire présente une forme générale d'ellipse partant du centre $O$ de la région cible et s'étendant vers l'extérieur de cette zone. En outre, l'unité de contrôle 50 commande les moyens générateurs d'énergie 20 pour qu'ils appliquent, en chaque point de la pluralité de points de tir de la trajectoire, une quantité d'énergie donnée. La forme en spirale de la trajectoire permet un dépôt d'énergie partant du centre $O$ de la région cible 60 et s'éloignant progressivement vers les bords de la région cible. On comprend qu'une telle trajectoire en spirale favorise la diffusion thermique depuis le centre de la région cible vers les bords de cette zone et permet de tirer partie de cette diffusion pour contrôler l'élévation de température dans la région cible.

Dans un repère ($Ox$,$Oz$) ayant pour origine le centre $O$ de la région cible 60, l'équation paramétrique de la trajectoire du point focal P peut être définie de la manière suivante :

$$\begin{cases} x(\xi) = \Delta a \cdot \dfrac{\xi}{2\pi} \cdot \cos(\xi) \\[2em] z(\xi) = \Delta b \cdot \dfrac{\xi}{2\pi} \cdot \sin(\xi) \end{cases} \qquad\qquad [1]$$

où ($x$,$z$) sont les coordonnées du point focal dans le repère ($Ox$,$Oz$), $\Delta a$ et $\Delta b$ sont les espacements des tours successifs respectivement suivants les axes $O_x$ et $Oz$ et $\xi$ est le paramètre de la trajectoire compris entre 0 et $2\pi \cdot N$, $N$ étant le nombre de tours de l'ellipse.

Au cours de la première trajectoire, le point focal P est déplacé de sorte que l'aire $\Omega$ ajoutée par unité de temps à la région déjà traitée est constante. Cette condition se traduit par l'équation différentielle suivante par rapport au temps $t$ :

$$\xi \cdot d\xi = \frac{2\pi \cdot \Omega}{\varepsilon \cdot (\Delta a)^2} dt \qquad\qquad [2]$$

où $\Omega$ est l'aire ajoutée par unité de temps à la région déjà couverte par la trajectoire en spirale et $\varepsilon$ vaut $^{\Delta b}/_{\Delta a}$ et définit l'excentricité de l'ellipse.

Cette première trajectoire elliptique est constituée d'une pluralité de points de tir discrets i, i allant de 0 à n, dont les positions par rapport au centre $O$ de l'ellipse sont définies par les vecteurs $r_i = (x_i,z_i)$ où $i \in [0,n]$.

Le point focal se déplace d'une position de tir i de position $r_i$ à la suivante i+1 de position $r_{i+1}$ à une cadence régulière. La durée de tir (application des ultrasons) $\Delta t$ est constante d'un point de tir à l'autre.

Selon une deuxième étape 200 , l'appareil d'imagerie mesure la distribution de température obtenue $T_1(x,z)$ dans la région cible.

Dans l'hypothèse où les tissus de la région cible seraient parfaitement homogènes, c'est-à-dire que les caractéristiques d'absorption et de diffusion de chaleur seraient uniformes dans l'ensemble de la région cible, la trajectoire réalisée au cours de la première étape 100 conduirait à une augmentation uniforme de la température dans la région cible.

Or ce n'est pas le cas car les tissus traités ne sont généralement pas homogènes.

La distribution de température mesurée permet de déterminer un coefficient de diffusion thermique moyen $D$.

Selon une troisième étape 300, l'unité de contrôle détermine en fonction de la distribution spatiale de température mesurée $T_1(x,z)$ une deuxième trajectoire (j=2). Cette deuxième trajectoire présente la même forme en spirale que la première trajectoire. Cependant, la vitesse de déplacement du point focal est modifiée selon une fonction de répartition $R_2$ de l'énergie qui dépend de la position $(x,z)$ du point focal sur la trajectoire, de sorte que :

$$\Re_2(x(\xi),z(\xi)) \cdot \xi \cdot d\xi = \frac{2\pi \cdot \Omega}{\varepsilon \cdot (\Delta a)^2} dt \qquad [3]$$

La fonction de répartition $R_2$ a pour effet de modifier la distance entre deux points de tirs successifs et par conséquent, la vitesse de déplacement du point focal le long de la trajectoire en spirale. Il convient de remarquer que l'équation [3] se réduit à l'équation [2] lorsque $R_2$ est remplacée par $R_1 = 1$ sur toute la région cible. La fonction $R_2$ est déterminée de manière à compenser les inhomogénéités persistantes après la première trajectoire en modulant la densité d'énergie délivrée localement dans la région cible.

La durée de chaque tir individuel est toujours de $\Delta t$, de sorte que le point focal se déplace d'un point de tir à l'autre à la même cadence que lors de la première trajectoire. Le nombre n+1 de tirs individuels le long de la trajectoire en spirale est toujours le même.

Selon une quatrième étape 400, l'unité de contrôle commande le système de déplacement pour positionner le point focal des moyens générateurs d'énergie successivement en une pluralité de points de tir de la deuxième trajectoire.

Les étapes 200, 300 et 400 qui précèdent sont éventuellement renouvelées pour réaliser un nombre M de trajectoires successives, de manière à imposer à la zone à traiter un profil de température de consigne pendant une durée de traitement prédéterminée. Pour chaque j-ième trajectoire, l'unité de contrôle détermine une nouvelle fonction de répartition $R_j$ et commande le système de déplacement pour faire évoluer le point focal des moyens générateurs d'énergie selon la j-ième trajectoire ainsi déterminée.

On va maintenant décrire de manière générale, l'étape de calcul 300 de la fonction de répartition $R_j$ réalisée par l'unité de contrôle.

On considère que le module de contrôle commande le système de déplacement et les moyens générateurs d'énergie pour que le point focal des moyens générateurs d'énergie parcoure un nombre M de trajectoires d'une forme prédéterminée.

On note :

$O$ un point de référence dans la région cible, par exemple situé au centre de la région cible,
$(Ox, Oz)$ un repère bidimensionnel d'origine $O$,
$\theta_j(x,z)$ la température de consigne à atteindre en un point $(x,z)$ lors du parcours de la j-ième trajectoire,
$T_j(x,z)$ la température effectivement mesurée au point $(x,z)$ par l'appareil d'imagerie IRM après le parcours de la j-ième trajectoire,
$R_j$ est la fonction de répartition déterminée pour la j-ième trajectoire,
$G(D,\tau)$ est la fonction de Green qui permet d'évaluer une variation de température due à la diffusion thermique $D$ dans les tissus (évaluée après la première trajectoire) durant une période de temps $\tau$ écoulée entre deux trajectoires.

La fonction de Green est par exemple décrite en annexe dans le document « Local hyperthermia with MR-guided focused ultrasound : Spiral trajectory of the focal point optimized for temperature uniformity in the target region », Journal of magnetic resonance imaging 12 :571-583 (publié en 2000). Elle a pour expression:

$$G(D,\sigma)(r,r') = \frac{1}{2 \cdot \pi \cdot \sqrt{2 \cdot D \cdot \tau}} \cdot \exp\left(-\frac{\|r - r'\|^2}{4 \cdot D \cdot \tau}\right)$$

L'efficacité du chauffage lors de la première trajectoire peut être exprimée sous la forme d'un coefficient $\alpha_1$ au point $O$ :

$$\alpha_1 = \frac{\theta_1(x=0, z=0)}{T_1(x=0, z=0)}$$

Le profil spatial de température de consigne est donné par :

$$\theta_{j+1}(r) = \eta_{j+1} \cdot \frac{\theta_1(r)}{\alpha} \text{ où } \alpha \prec \eta_{j+1} \leq 1 \qquad [4]$$

Dans cette équation, la valeur de $\eta_{j+1}$ définit l'évolution souhaitée de la température entre la j-ième et la (j+1)-ième trajectoire. $\eta_{j+1} = 1$ correspond à une température stationnaire dans la région cible. C'est-à-dire que le dépôt d'énergie dans la région cible lors de la (j+1)-ième trajectoire, défini par la fonction de répartition $R_{j+1}$, doit uniquement compenser les pertes de chaleur dues à la conduction thermique dans les tissus.

La valeur de $\eta_j$ est fixé pour chaque trajectoire j à l'aide d'un algorithme itératif, à la valeur maximale (comprise entre $\alpha$ et 1) pour laquelle la puissance requise fournie par les moyens générateurs d'énergie ne dépasse pas une valeur limite de tolérance technique des instruments. La valeur limite de tolérance dépend des moyens générateurs d'énergie, cette valeur limite est définie par le fournisseur de ces moyens générateurs d'énergie.

La température en un point $r(x,z)$ qui sera obtenue après le parcours de la (j+1)-ième trajectoire peut être évaluée de la manière suivante :

$$T_{j+1}(r) = \left[ T_j \otimes G(D,\tau) \right](r) + \Re_{j+1}(r) \cdot \theta_1(r) \qquad [5]$$

Cette température prend en compte la diffusion thermique entre la j-ième et la (j+1)-ième trajectoire et la nouvelle répartition $R_{j+1}$ de l'énergie. La fonction de Green est toujours la même dans la mesure où le coefficient de diffusion thermique $D$ est supposé constant et que la durée $\tau$ est la même à chaque trajectoire.

Une condition de l'asservissement est : $T_{j+1} = \theta_{j+1}$

On en déduit :

$$\Re_{j+1}(r) \cdot \theta_1(r) = \theta_{j+1}(r) - \left[ T_j \otimes G(D,\tau) \right](r) \qquad [6]$$

$$\Re_{j+1}(r) \cdot \theta_1(r) = \left[ Tj(r) - \left[ T_j \otimes G(D,\tau) \right](r) \right] + \left[ \theta_{j+1}(r) - \theta_j(r) \right]$$
$$+ \left[ \theta_j(r) - T_j(r) \right] \qquad [7]$$

Cette équation est l'équation centrale d'une loi de régulation de type différentielle et proportionnelle. Dans le cadre de l'invention, cette expression est modifiée pour obtenir une loi de régulation PID (Proportionnelle Intégrale et Différentielle). L'équation de cette loi de régulation PID est alors :

$$\mathfrak{R}_{j+1}(r) = \underbrace{\left[ T_j(r) - \left[ T_j \otimes G(D,\tau) \right](r) \right]}_{(1)} + \underbrace{\left[ \theta_{j+1}(r) - \theta_j(r) \right]}_{(2)}$$

$$+ a \cdot \underbrace{\left[ \theta_j(r) - T_j(r) \right]}_{(3)} + \frac{a^2}{4} \cdot \underbrace{\sum_{k=0}^{j} \left[ \theta_j(r) - T_j(r) \right]}_{(4)} \qquad [8]$$

Dans l'équation [8], le premier terme (1) tient compte de la variation de température due à la diffusion de chaleur dans les tissus entre deux trajectoires successives j et j+1. Le deuxième terme (2) est le terme différentiel de la loi de régulation qui tient compte de la couche supplémentaire ajoutée au profil de température par la (j+1)-ième trajectoire. Le troisième terme (3) est le terme proportionnel de la loi de régulation qui tient compte de l'erreur instantanée entre la température mesurée et la température de consigne définies pour la j-ième trajectoire précédente. Enfin, le quatrième terme (4) est le terme intégral de la loi de régulation qui tient compte des erreurs entre la température mesurée et la température de consigne définies pour chacune des trajectoires antérieures. Le paramètre $a$ est une grandeur adimensionnelle qui est liée au temps de réponse de la boucle d'asservissement du système de déplacement des moyens générateur d'énergie. Le temps de réponse de la boucle d'asservissement est $2\tau/_a$.

Il convient de noter que plus le paramètre $a$ est grand, plus l'asservissement devient sensible au bruit expérimental avec de possibles fluctuations. Une valeur recommandée pour le paramètre $a$ est de $2\left(\sqrt{2} - 1\right) \approx 0{,}8284$. En effet, cette valeur conduit à l'élimination des termes (3) et (4) dans l'équation [8] lors du calcul de $R_2$ après le parcours de la première trajectoire. Le temps de réponse de la boucle d'asservissement est alors de $2\tau/_a \approx 2{,}4143 \cdot \tau$, ce qui représente le temps nécessaire à l'asservissement pour rattraper une éventuelle erreur dans la température mesurée.

L'équation [8] équivaut à :

$$\mathfrak{R}_{j+1}(r) \cdot \theta_1(r) = \theta_{j+1}(r) - \left[ T_j \otimes G(D,\tau) \right](r) - (1-a) \cdot \left[ \theta_j(r) - T_j(r) \right]$$

$$+ \frac{a^2}{4} \cdot \sum_{k=0}^{j} \left[ \theta_k(r) - T_k(r) \right] \qquad [9]$$

Pour que la durée d'une trajectoire soit toujours constante, la trajectoire peut être dilatée ou comprimée dans le temps pour ramener sa durée à la valeur $\tau$. Simultanément la puissance ultrasonore déposée par les moyens générateurs d'énergie est renormalisée dans le sens inverse par un facteur qui est égal à la moyenne spatiale de $R_{j+1}$ dans la région d'intérêt.

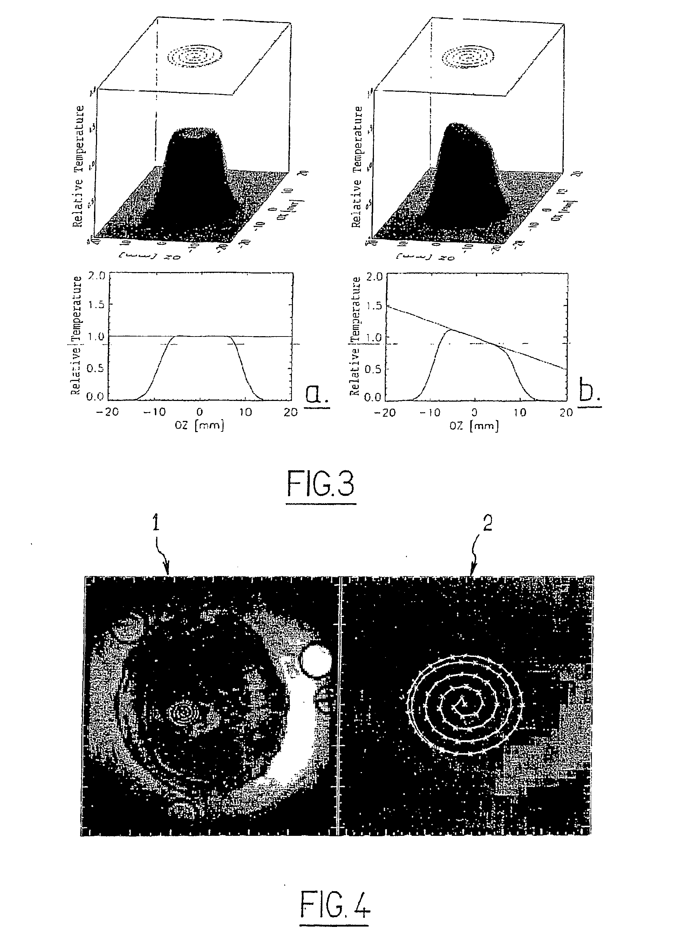

La figure 3 représente des exemples a et b de profils spatiaux $R_j(r) \cdot \theta_1(r)$ de température de consigne tels qu'ils peuvent être commandés dans le cas d'une trajectoire en forme de spirale. Ces exemples sont basés sur des trajectoires en forme de spirale présentant des diamètres de 12 mm et de 16 mm respectivement suivant les axes $Ox$ et $Oz$. Le coefficient de diffusion $D$ est de 0,05 mm$^2$/s. La trajectoire comprend $n+1=100$ points de tir, les tirs étant espacés de $\Delta t$ = 1,6s. L'exemple a correspond au cas d'une fonction de répartition uniforme $R_1(x,z) = 1$ tandis que l'exemple b correspond à une fonction de répartition $R_j$ présentant une pente constante (gradient constant suivant l'axe $Ox$ de - 0,025 mm$^{-1}$.

La figure 4 représente un exemple de trajectoire en forme de spirale elliptique parcourue par le point focal des moyens générateurs d'énergie. Le champ de vue est de 128$\times$128 mm$^2$ sur l'image 1 tandis que l'image 2 a été agrandie avec un taux d'agrandissement de 4. La trajectoire en forme de spirale présente des diamètres de 15 mm et 11 mm respectivement suivant les axes $Ox$ et $Oz$. L'image d'arrière-plan est obtenue à l'aide de la séquence d'échos de gradient utilisée pour la thermométrie par IRM. Trois échantillons de gel d'agar sont visibles sur l'image 1. Ces échantillons permettent la correction en trois points de la thermométrie. Des inhomogénéités sont visibles dans la structure de l'échantillon traité.

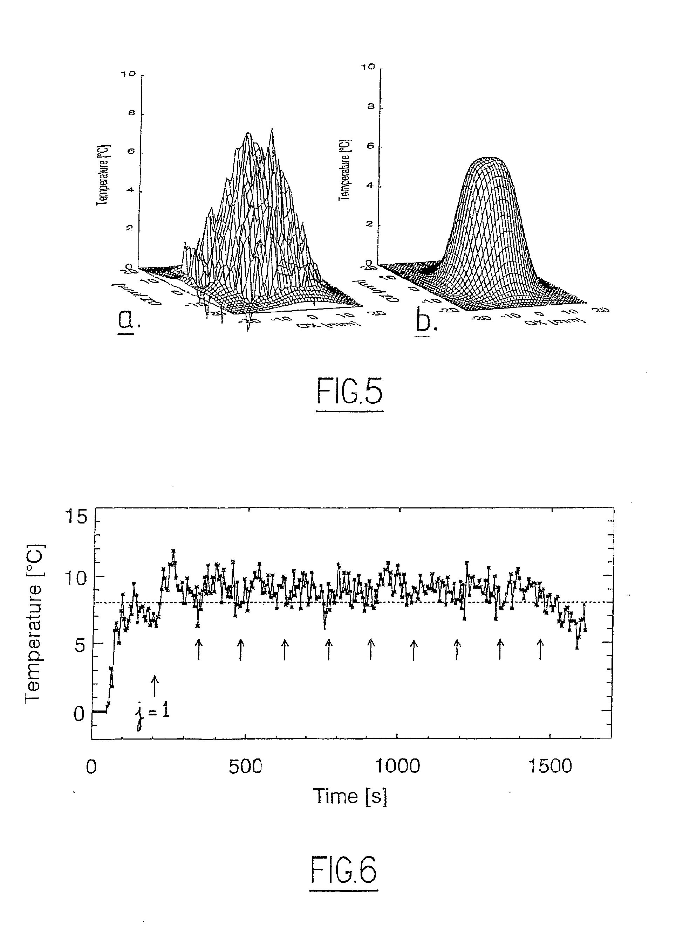

Sur la figure 5, le diagramme a représente la distribution de température mesurée après que le point focal des moyens générateurs d'énergie ait parcouru la trajectoire en forme de spirale représentée sur la figure 4. Le diagramme b représente la distribution de température simulée pour un coefficient de diffusion thermique uniforme $D = 0{,}13$ mm$^2$/s

des tissus à traiter. Le diagramme b est le diagramme qui se rapproche le plus du profil expérimental du diagramme a. Ce diagramme b permet d'estimer le coefficient de diffusion thermique moyen dans la région cible à traiter.

La figure 6 est une courbe de l'évolution de la température mesurée au centre $O$ de la région cible en fonction du temps lors d'une expérimentation ex vivo. La température mesurée évolue au cours d'une série de $M = 10$ trajectoires successives. La température de consigne est de 8°C dans toute la région cible (régime d'hyperthermie sous-létale).

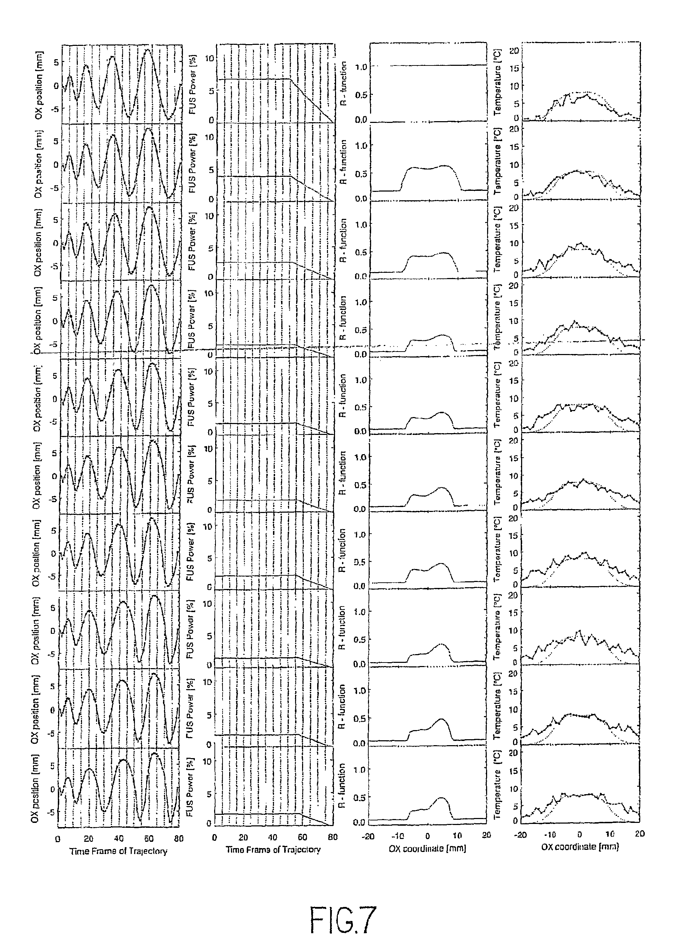

La figure 7 représente sur une première colonne, l'évolution de la coordonnée $x$ du point focal de chacune des dix trajectoires de la série réalisée lors de l'expérimentation de la figure 6. La deuxième colonne représente l'évolution de la puissance ultrasonore déposée par les moyens générateurs d'énergie au cours de chacune des trajectoires. La troisième colonne représente le profil de température mesurée dans une coupe de la région cible passant par le point $O$, centre de la région cible, après chaque trajectoire. La quatrième colonne représente le profil de montée en température mesurée dans la coupe de la région cible, obtenue au cours de chaque trajectoire.

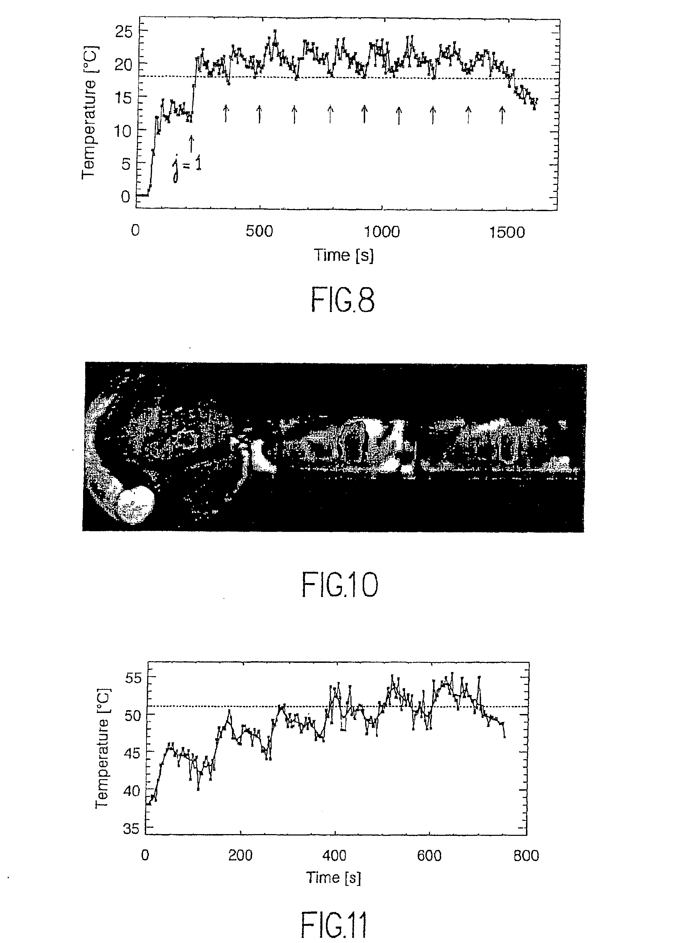

La figure 8 est une courbe de l'évolution de la température mesurée au centre $O$ de la région cible en fonction du temps lors d'une expérimentation ex vivo. La température mesurée évolue au cours d'une série de $M = 10$ trajectoires successives. La température de consigne est supérieure de 18°C à la température initiale dans toute la région cible (régime d'hyperthermie létale) en vue de réaliser une ablation thermique.

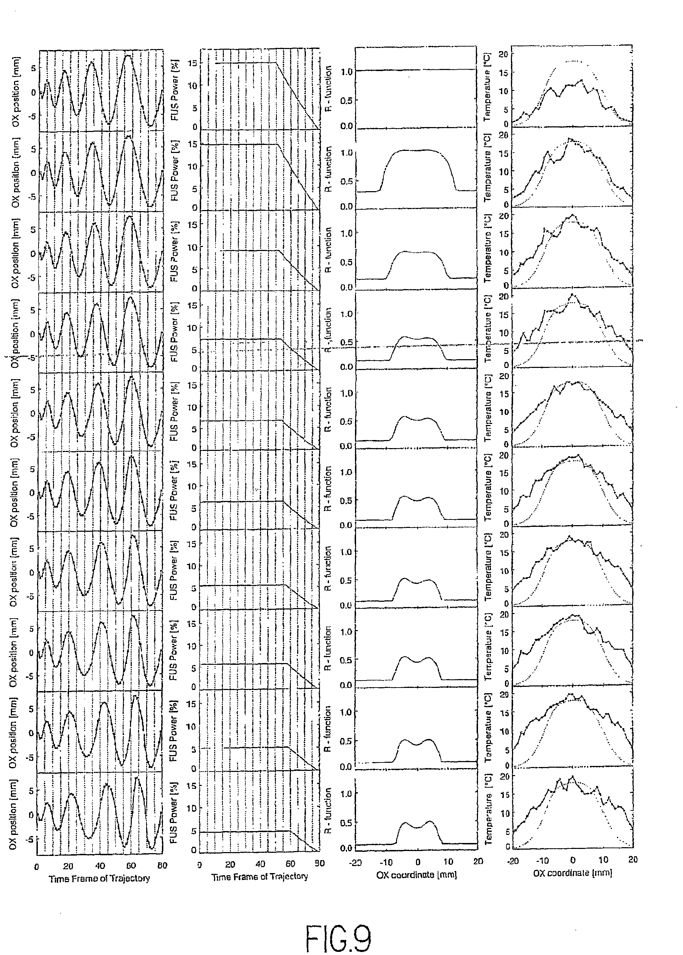

La figure 9 représente sur une première colonne, l'évolution de la coordonnée $x$ du point focal de chacune des dix trajectoires de la série réalisée lors de l'expérimentation de la figure 8. La deuxième colonne représente l'évolution de la puissance ultrasonore déposée par les moyens générateurs d'énergie au cours de chacune des trajectoires. La troisième colonne représente le profil de la fonction de répartition R calculé selon une direction passant par le point $O$, centre de la région cible, après chaque trajectoire. La quatrième colonne représente le profil de montée en température mesurée dans la coupe de la région cible, obtenue au cours de chaque trajectoire.

La figure 10 est un exemple de carte de température tridimensionnelle obtenue par un appareil d'imagerie IRM à la fin de la sixième trajectoire correspondant à la figure 11. Les données expérimentales ont été obtenues avec une résolution spatiale de $1 \times 1 \times 5$ mm$^3$ et lissées par convolution avec une gaussienne bidimensionnelle d'écart-type égal à 1 mm dans chaque direction ($Ox$) et ($Oz$). La distribution de température ne peut pas contenir de hautes fréquences spatiales à cause de la diffusion thermique.

La figure 11 représente une courbe de l'évolution de la température au centre $O$ de la région cible en fonction du temps lors d'une expérimentation in vivo (diamètre de la région cible de 11 mm). La température mesurée évolue au cours d'une série de $M = 6$ trajectoires successives. La ligne en traits pointillés représente la consigne de montée en température (13 degrés Celsius au-dessus de la température physiologique). L'écart entre deux trajectoires successives est de 2 minutes.

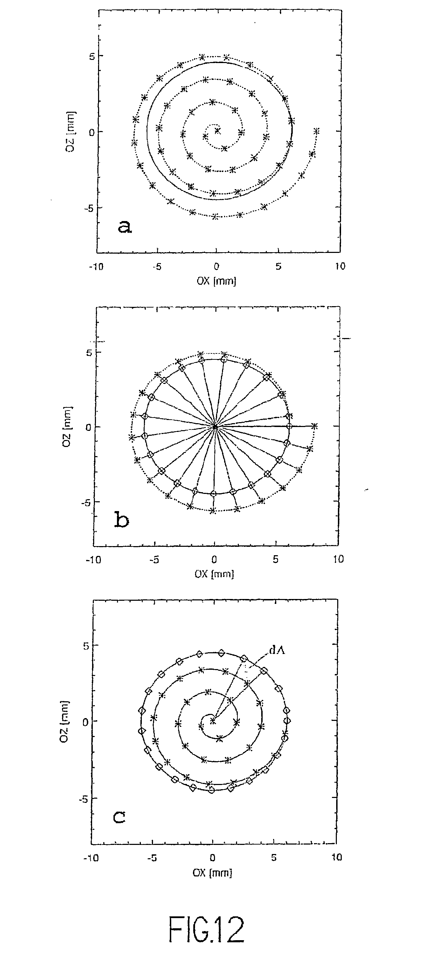

Sur la figure 12, le diagramme a représente les positions des différents points de tir le long de la première trajectoire (j=1) et les limites de la région cible à traiter (ellipse). Comme on peut le voir sur cette figure, la trajectoire en spirale recouvre progressivement la région cible. La trajectoire comprend en outre un tour supplémentaire qui s'étend hors de la région cible. Ce tour supplémentaire n'est en fait pas exécuté réellement par les moyens générateurs d'énergie. Il sert uniquement à calculer la quantité d'énergie à déposer au niveau de la frontière de la région cible.

Sur la figure 12, le diagramme b représente une étape consistant à projeter les points de tir du tour supplémentaire sur le contour de la région cible (ellipse) selon des directions de projection radiales.

Sur la figure 12, le diagramme c représente la trajectoire avec les points de tir successifs tels qu'elle sera réellement exécutée par les moyens générateurs d'énergie. Cette trajectoire réelle comprend les points de tir du tour supplémentaire projetés sur le contour de la région cible.

## Revendications

1. Ensemble (1) de traitement thermique d'une région (60) d'un tissu biologique comprenant :

    - des moyens générateurs d'énergie (20) pour fournir de l'énergie en un point focal (P) dans la région,
    - des moyens (10) pour mesurer la distribution spatiale de température dans ladite région,
    - une unité de contrôle (40) comprenant des moyens aptes à commander le déplacement du point focal (P) le long d'une trajectoire (j) prédéterminée en vue d'obtenir une distribution spatiale de température conforme à une distribution de consigne,
    l'unité de contrôle (40) comprend des moyens qui, au cours du déplacement du point focal (P), sont aptes à commander la répartition d'énergie fournie par les moyens générateurs (20) le long de la trajectoire, en fonction de la distribution de température mesurée et de la distribution de consigne, selon une loi de régulation comprenant un terme Proportionnel-Intégral-Dérive, et **caractérisé en ce que**,
    pour commander une répartition d'énergie, l'unité de contrôle (40) comprend des moyens aptes à déterminer une fonction de répartition ($R_j$) définissant la position du point focal (P) le long de la trajectoire en fonction du temps.

2. Ensemble selon la revendication 1, dans lequel la loi de régulation prend en compte un coefficient de diffusion (*D*) thermique de la région cible.

3. Ensemble selon l'une des revendications 1 ou 2, dans lequel l'unité de contrôle (40) comprend des moyens aptes à commander le déplacement du point focal (P) le long d'une série de trajectoires (j) successives et à commander pour chaque trajectoire une répartition d'énergie correspondante, en fonction d'une distribution de consigne associée à cette trajectoire et des distributions de température mesurées au cours des trajectoires précédentes.

4. Ensemble selon l'une des revendications 1 à 3, dans lequel l'unité de contrôle (40) comprend des moyens aptes à commander le déplacement du point focal (P) en une pluralité de points de tir (i) discrets répartis le long de la trajectoire.

5. Ensemble selon la revendication 4, dans lequel l'unité de contrôle (40) comprend des moyens aptes à commander les moyens générateurs d'énergie (20) pour qu'ils déposent en chaque point de tir (i) une quantité donnée d'énergie, égale d'un point de tir à l'autre.

6. Ensemble selon l'une des revendications 1 à 5, dans lequel l'unité de contrôle (40) comprend des moyens aptes à commander le déplacement du point focal (P) le long d'une série de trajectoires successives et à commander pour chaque trajectoire (j) une répartition d'énergie ($R_j$) correspondante telle que :

$$\Re_{j+1}(r) \cdot \theta_1(r) = \theta_{j+1}(r) - \left[ T_j \otimes G(D,\tau) \right](r) - (1-a) \cdot \left[ \theta_j(r) - T_j(r) \right]$$

$$+ \frac{a^2}{4} \cdot \sum_{k=0}^{j} \left[ \theta_k(r) - T_k(r) \right]$$

où r est la position d'un point dans la région cible, $\theta_1$ est la distribution de température de consigne à atteindre lors du parcours de la première trajectoire, $\theta_j$ est la distribution de température de consigne à atteindre lors du parcours de la j-ième trajectoire, $T_j$ est la distribution de température effectivement mesurée par les moyens de mesure après le parcours de la j-ième trajectoire,
$G(D,\tau)$ est la fonction de Green qui dépend de la diffusion thermique *D* dans la région cible durant une période de temps τ écoulée entre deux trajectoires j et j+1
*a* est un paramètre adimensionnel d'asservissement.

7. Ensemble selon l'une des revendications qui précèdent, dans lequel l'unité de contrôle (40) comprend des moyens aptes à déplacer le point focal (P) le long d'une trajectoire en forme générale de spirale.

8. Ensemble selon l'une des revendications qui précèdent, dans lequel l'unité de contrôle (40) comprend des moyens aptes à commander le déplacement du point focal (P) en fonction d'une distribution de température de consigne uniforme dans la région cible (60).

9. Ensemble selon l'une des revendications qui précèdent, dans lequel l'unité de contrôle (40) comprend des moyens aptes à commander le déplacement du point focal (P) en fonction d'une distribution de température de consigne présentant un gradient uniforme selon une direction, dans la région cible.

**Patentansprüche**

1. Einheit (1) zur thermischen Behandlung eines Bereichs (60) eines biologischen Gewebes, umfassend:

- Mittel zur Erzeugung von Energie (20), um Energie in einen Brennpunkt (P) des Bereichs zu liefern
- Mittel (10), um die räumliche Verteilung in dem Bereich zu messen.
- eine Steuerungseinheit (40), umfassend Mittel, die ausgelegt sind, um die Verschiebung des Brennpunkts (P) entlang einer vorbestimmten Bahn (j) zu steuern, um eine räumliche Verteilung der Temperatur gemäß einer Vorgabe-Verteilung zu erhalten,

wobei die Steuerungseinheit (40) Mittel umfasst, die, im Laufe der Verschiebung des Brennpunkts (P), ausgelegt sind, um die Verteilung von Energie, die von den Mitteln zur Erzeugung (20) geliefert wird, entlang der Bahn zu steuern, je nach der gemessenen Verteilung der Temperatur und der Vorgabe-Verteilung, gemäß einem Regulierungsgesetz, umfassend einen poroportional-integral-abgeleiteten Ausdruck, und **dadurch gekennzeichnet, dass**,

um eine Verteilung von Energie zu steuern, die Steuerungseinheit (40) Mittel umfasst, die ausgelegt sind, um eine Verteilungsfunktion ($R_j$) zu bestimmen, die die Position des Brennpunkts (P) entlang der Bahn als eine Funktion der Zeit definiert.

2. Einheit nach Anspruch 1, wobei das Regulierungsgesetz einen Koeffizienten der thermischen Verteilung ($D$) des Zielbereichs in Betracht zieht.

3. Einheit nach einem der Ansprüche 1 oder 2, wobei die Steuerungseinheit (40) Mittel umfasst, die ausgelegt sind, um die Verschiebung des Brennpunkts (P) entlang einer Reihe von aufeinander folgenden Bahnen (j) zu steuern und für jede Bahn eine entsprechende Energieverteilung zu steuern, je nach einer Vorgabe-Verteilung, die mit dieser Bahn assoziiert ist, und Temperaturverteilungen, die im Laufe der vorhergehenden Bahnen gemessen wurden.

4. Einheit nach einem der Ansprüche 1 bis 3, wobei die Steuerungseinheit (40) Mittel umfasst, die ausgelegt sind, um die Verschiebung des Brennpunkts (P) an einer Vielzahl von diskreten Abschusspunkten (i) zu steuern, die entlang der Bahn verteilt sind.

5. Einheit nach Anspruch 4, wobei die Steuerungseinheit (40) Mittel umfasst, die ausgelegt sind, um die Mittel zur Erzeugung von Energie (20) zu steuern, damit sie an jedem Abschusspunkt (i) eine bestimmte Menge von Energie ablegen, gleich an allen Abschusspunkten.

6. Einheit nach einem der Ansprüche 1 bis 5, wobei die Steuerungseinheit (40) Mittel umfasst, die ausgelegt sind, um die Verschiebung des Brennpunkts (P) entlang einer Reihe von aufeinander folgenden Bahnen zu steuern und für jede Bahn (j) eine entsprechende Energieverteilung ($R_j$) zu steuern, wie

$$\Re_{j+1}(r) \cdot \theta_1(r) = \theta_{j+1}(r) - \left[T_j \otimes G(D,\tau)\right](r) - (1-a) \cdot \left[\theta_j(r) - T_j(r)\right] + \frac{a^2}{4} \cdot \sum_{k=0}^{j} \left[\theta_k(r) - T_k(r)\right]$$

wobei $r$ die Position eines Punkts im Zielbereich ist, $\theta_1$ die Vorgabe-Temperaturverteilung ist, die beim Weg der ersten Bahn erreicht werden soll, $\theta j$ die Vorgabe-Temperaturverteilung ist, die beim Weg der j-ten Bahn erreicht werden soll, $Tj$ die Temperaturverteilung ist, die von den Mitteln zur Messung nach dem Weg der j-ten Bahn effektiv gemessen wird,

$G(D,\tau)$ die Green-Funktion ist, die von der thermischen Diffusion $D$ im Zielbereich während des Zeitraums I abhängt, der zwischen zwei Bahnen j und j+1 1 vergeht,

a ein dimensionsloser Regelparameter ist.

7. Einheit nach einem der vorhergehenden Ansprüche, wobei die Steuerungseinheit (40) Mittel umfasst, die ausgelegt sind, um den Brennpunkt (P) entlang einer Bahn mit im Allgemeinen einer Spiralform zu verschieben.

8. Einheit nach einem der vorhergehenden Ansprüche, wobei die Steuerungseinheit (40) Mittel umfasst, die ausgelegt sind, um die Verschiebung des Brennpunkts (P) je nach einer gleichförmigen Vorgabe-Temperaturverteilung im Zielbereich (60) zu steuern.

9. Einheit nach einem der vorhergehenden Ansprüche, wobei die Steuerungseinheit (40) Mittel umfasst, die ausgelegt sind, um die Verschiebung des Brennpunkts (P) je nach einer Vorgabe-Temperaturverteilung, die einen gleichförmigen Gradienten in einer Richtung aufweist, im Zielbereich zu steuern.

**Claims**

1. Assembly (1) for the heat treatment of a region (60) of a biological tissue comprising:

- energy-generating means (20) for supplying energy at a focal point (P) in the region,
- means (10) for measuring the spatial temperature distribution in said region,
- a control unit (40) comprising means able to control the movement of the focal point (P) along a predetermined path (j) with a view to obtaining a spatial temperature distribution in accordance with a set distribution, the control unit (40) comprises means which, during the movement of the focal point (P) are able to control the energy distribution supplied by the generating means (20) along the path, according to the measured temperature distribution and the set distribution in accordance with a regulation law comprising a proportional-integral-derivative term, **characterised in that**, in order to control an energy distribution, the control unit (40) comprises means able to determine a distribution function ($R_j$) defining the position of the focal point (P) along the path as a function of time.

2. Assembly according to claim 1, in which the regulation law takes into account a coefficient of thermal diffusion (D) of the target region.

3. Assembly according to one of claims 1 or 2, in which the control unit (40) comprises means able to control the movement of the focal point (P) along a series of successive paths and to control, for each path, a corresponding energy distribution, according to a set distribution associated with this path and temperature distributions measured during the previous paths.

4. Assembly according to one of claims 1 to 3, in which the control unit (40) comprises means able to control the movement of the focal point (P) at a plurality of discreet shooting points (i) distributed along the path.

5. Assembly according to claim 4, in which the control unit (40) comprises means able to control the energy-generating means (20) so that they deposit, at each shooting point (i), a given quantity of energy, equal from one shooting point to the other.

6. Assembly according to one of claims 1 to 5, in which the control unit (40) comprises means able to control the movement of the focal point (P) along a series of successive paths and to control, for each path (j), a corresponding energy distribution ($R_j$) such that:

$$\Re_{j+1}(r) \cdot \theta_1(r) = \theta_{j+1}(r) - \left[T_j \otimes G(D,\tau)\right](r) - (1-a) \cdot \left[\theta_j(r) - T_j(r)\right] + \frac{a^2}{4} \cdot \sum_{k=0}^{j} \left[\theta_k(r) - T_k(r)\right]$$

where $r$ is the position of the point in a target region, $\theta_1$ is the set temperature distribution to be achieved when running over the first path, $\theta_j$ is the set temperature distribution to be achieved when running over the j$^{th}$ path, $T_j$ is the temperature distribution actually measured by the measurement means after running over the j$^{th}$ path, $G(D,\tau)$ is the Green's function that depends on the thermal distribution $D$ in the target region during a period of time $\tau$ that has elapsed between two paths j and j+1 $a$ is a dimensional control parameter.

7. Assembly according to one of the preceding claims, in which the control unit (40) comprises means able to move the focal point (P) along a path in the general form of a spiral.

8. Assembly according to one of the preceding claims, in which the control unit (40) comprises means able to control the movement of the focal point (P) according to a uniform set temperature distribution in the target region (60).

9. Assembly according to one of the preceding claims, in which the control unit (40) comprises means able to control the movement of the focal point (P) according to a set temperature distribution having a uniform gradient in one direction, in the target region.

FIG.1

FIG.2

FIG.3

FIG.4

FIG.5

FIG.6

FIG.7

FIG.8

FIG.10

FIG.11

FIG.9

FIG.12

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- FR 2798296

- FR 2823678

**Littérature non-brevet citée dans la description**

- Local hyperthemia with MR-guided focused ultrasound : Spiral trajectory of the focal point optimized for temperature uniformity in the target region. *Journal of magnetic résonance imaging,* 2000, vol. 12, 571-583

- Local hyperthermia with MR-guided focused ultrasound : Spiral trajectory of the focal point optimized for temperature uniformity in the target region. *Journal of magnetic resonance imaging,* 2000, vol. 12, 571-583